# EUROPEAN PATENT APPLICATION

(11) **EP 1 538 216 A1**
(43) Date of publication of application: **08.06.2005**
(21) Application number: 03795302.3
(22) Date of filing: 05.09.2003
(51) Int. Cl.: C12P 41/00, C07C 227/30, C07C 227/20, C07C 229/28, C07C 269/06, C07C 271/22, C07C 233/47

(54) **PROCESS FOR PRODUCING OPTICALLY ACTIVE ERYTHRO 3-CYCLOHEXYLSERINES**

(30) Priority: 09.09.2002 JP 2002262788
(71) Applicant: Nippon Kayaku Kabushiki Kaisha, Tokyo 102-8172 (JP)
(72) Inventor: IKEO, Takayoshi, Kawaguchi-shi, Saitama 332-0011 (JP); YAMAMOTO, Ken-ichi, Saitama-shi, Saitama 330-0835 (JP)
(74) Representative: Gille Hrabal Struck Neidlein Prop Roos
(86) International application number: PCT/JP2003/011390
(87) International publication number: WO 2004/024934

(57) **Abstract**

The present invention relates to a process in which N-acyl form of DL-erythro 3-substituted serines represented by the following general formula (1): 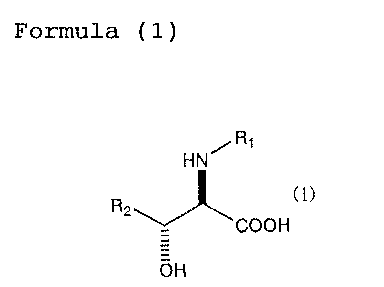 (wherein, R₁ means an alkanoyl group having 1-10 carbons, a benzoyl group, a halogen-substituted alkanoyl group having 1-5 carbons or a halogen-substituted benzoyl group, and R₂ means a phenyl group or a cyclohexyl group) is subjected to asymmetrical hydrolysis with L-aminoacylase or D-aminoacylase, thereby giving unreacted N-acyl-D-erythro 3-substituted serine in the case of L-aminoacylase or hydrolyzed D-erythro 3-substituted serine in the case of D-aminoacylase. The D-erythro forms are useful as an intermediate for medicines, for example, known to be useful as an anti-HIV drug (WO 01/40227).

## Description

### Technical Field

The present invention relates to a process for producing optically active erythro 3-cyclohexylserines which are useful as an intermediate for medicines and the like, for example, to a process for producing D-erythro 3-cyclohexylserine or a N-t-butoxycarbonylated form thereof (hereinafter, referred to as Boc form) which is an intermediate for medicines known to be useful as an anti-HIV drug (WO 01/40227).

### Background Art

J. Am. Chem. Soc. 1953, 75, p3417 and 1953, 75, p3421 disclose a process in which benzaldehyde and glycine are subjected to condensation reaction in the presence of a strong alkali to give DL-threo/erythro 3-phenylserines, and then the threo/erythro forms are subjected to mutual separation treatment. Since the process results in a low stereoselectivity of the reaction, 4 kinds of isomeres generate irreversibly according to combinations of threo/erythro and D/L and, in addition, the threo form generates in preferential to the erythro form. Therefore, upon separating L- or D-erythro 3-phenylserine, complicated steps are required for separating these isomers, resulting in generating a large problem in developing an industrial production process. In particular, one problem is that production ratio of the erythro form is smaller compared with that of the threo form. Further, in conventional optical resolution methods of D/L forms, there are such problems that an optical resolution agent used is expensive, and that, in addition, process and control thereof are complex with a low yield.

Other than the above process, a porcess employing asymmetric synthesis is known (J. Org. Chem. 1998, 63, 3631-3646). That is, it is a process in which the carboxylic acid moiety of L-serine as a stating material is protected with a protective group, followed by oxidizing the hydroxyl group to aldehyde, which is then subjected to Grignard asymmetric addition reaction thereby giving optically active erythro 3-cyclohexylserine. However, since the process needs very long steps and is of a low yield, it is difficult to say that the process is suitable for industrial production. As for a process carrying out optical resolution by utilizing an enzyme, a process is also known in which 3-cyclohexylserine of D form is decomposed by using D-threonine aldolase to obtain L-threo/erythro 3-cyclohexylserines, followed by decomposing L-erythro 3-cyclohexylserine enzymatically with L-allothreonine aldolase to obtain L-threo 3-cyclohexylserine (JP-A-6-125787). However, an aminoacylase that uses N-acyl-D- or L-erythro 3-substituted serine as a substrate and hydrolyzes the compound has not been known. Accordingly, a process that enables one to obtain optically active erythro 3-cyclohexylserine while using an enzyme has not been known.

Thus, development of an effective and industrial production process of optically active erythro 3-cyclohexylserines or Boc form thereof is expected.

### Disclosure of the invention

The inventors have intensively studied a process for obtaining optically active erythro 3-cyclohexylserines. As results, the inventors found that there exists L- or D-aminoacylase having the ability to hydrolyze selectively either N-acyl-D- or L-erythro 3-substituted serines, and that, by hydrolyzing either of them by allowing the enzyme to act on N-acyl-DL-erythro 3-phenylserines, targeted optically active erythro 3-phenylserines can be obtained with a high efficiency and high optical activity ,and completed the invention.

That is, the invention relates to:
(1) a process for producing N-acyl-D-erythro 3-substituted serine represented by the general formula (2): 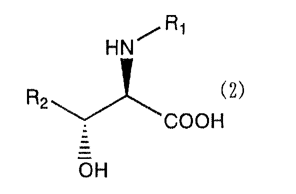 (in the formula (2), R₁ represents an acyl group and R₂ means a phenyl group or a cyclohexyl group), characterized by allowing L-aminoacylase having the ability of hydrolyzing selectively N-acyl-L-erythro 3-substituted serines to act on N-acyl form of DL-erythro 3-substituted serines represented by the general formula (1) to conduct asymmetrical hydrolysis: 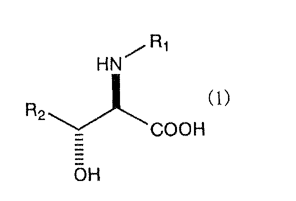 (in the formula (1), R₁ and R₂ represent the same meanings as those in the formula (2));
(2) the method described in the item (1), wherein the acyl group is an alkanoyl group having 1-10 carbons, a benzoyl group, a halogen-substituted alkanoyl group having 1-5 carbons or a halogen-substituted benzoyl group;
(3) the method described in the item (1), wherein R₁ is an alkanoyl group having 1-5 carbons;
(4) the method described in the item (3), wherein R₁ is an acetyl group;
(5) a process for producing D-erythro 3-substituted serine represented by the general formula (3): 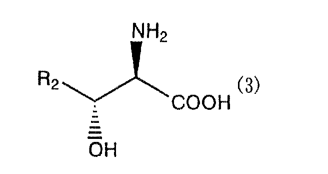 (in the formula (3), R₂ means a phenyl group or a cyclohexyl group), characterized by allowing D-aminoacylase having the ability of hydrolyzing selectively N-acyl-D-erythro 3-substituted serines to act on N-acyl form of DL-erythro 3-substituted serines represented by the general formula (1) to conduct asymmetrical hydrolysis: 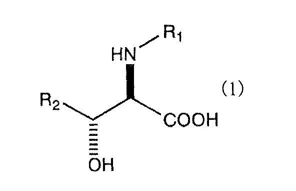 (in the formula (1), represents an acyl group, R₂ means a phenyl group or a cyclohexyl group);
(6) the method described in the item (5), wherein the acyl group is an alkanoyl group having 1-10 carbons, a benzoyl group, a halogen-substituted alkanoyl group having 1-5 carbons or a halogen-substituted benzoyl group;
(7) the method described in the item (5), wherein R₁ is an alkanoyl group having 1-5 carbons;
(8) the production method described in the item (7), wherein R₁ is an acetyl group;
(9) a process for producing D-erythro 3-cyclohexylserine represented by the following formula (E): 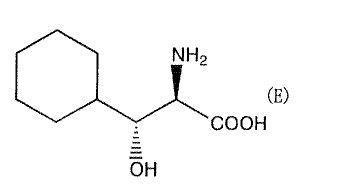 characterized by subjecting N-acyl-D-erythro 3-phenylserine represented by the following formula (C): 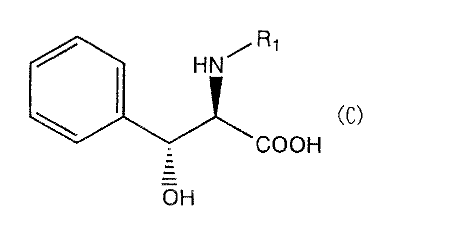 (in the formula (C), R₁ represents an acyl group) obtained by the process according to any one of preceding items (1) to (4) to catalytically hydrogen reduction with rhodium-carbon to give N-acyl-D-erythro 3-cyclohexylserine represented by the following formula (D): 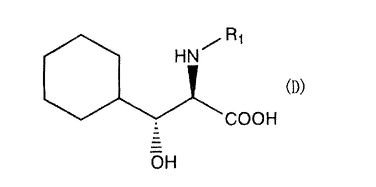 (in the formula (D), R₁ represents the same meaning as that in the formula (C)), and by further hydrolyzing a compound of the above formula (D);
(10) a process for producing N-t-butoxycarbonyl-D-erythro 3-cyclohexylserine represented by the following formula (F): 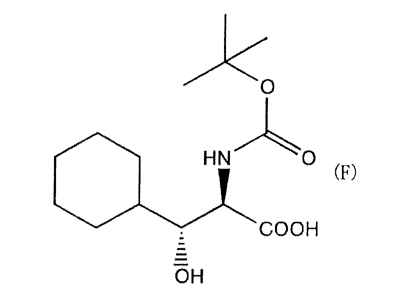 characterized by converting D-erythro 3-phenylserine represented by the following formula (G): 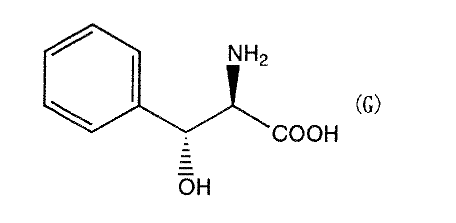 obtained by the process according to any one of preceding items (5) to (8) into Boc form with di-t-butyldicarbonate to give N-t-butoxycarbonyl-D-erythro 3-phenylserine represented by the following formula (H): 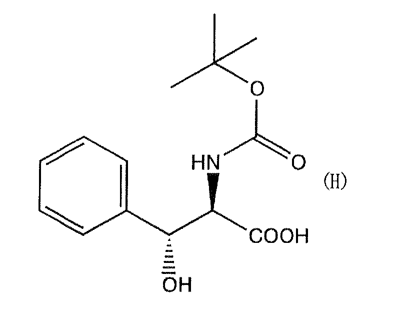 and by further subjecting a compound of the above formula (H) to catalytically hydrogen reduction with rhodium-carbon; and (11) N-acetyl-D-erythro 3-cyclohexylserine represented by the following formula (D)':

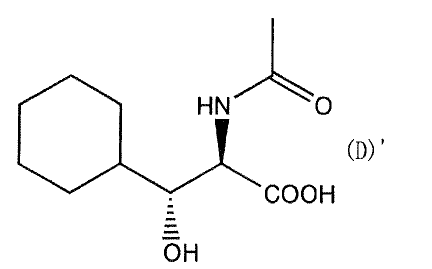

### Best Mode for Carrying Out the Invention

Hereinafter, the invention will be described in detail.

The N-acyl-DL-erythro 3-substituted serines of the above general formula (1) for use in the invention may be obtained, for example, according to the process described in J. Am. Chem. Soc. 1953, 75, p238, and 1953, 75, p89.

L- or D-aminoacylase for use in the invention having the ability of hydrolyzing selectively either N-acyl-D- or
- L-erythro 3-substituted serines is not particularly limited as long as it has the ability of hydrolyzing selectively either of N-acyl groups contained in the D form and the L form of the N-acyl form represented by the general formula (1). For example, it may be either an animal origin or microorganisms origin. The L-aminoacylase that has the ability to hydrolyze selectively the L form of the N-acyl form represented by the general formula (1) can be used without particular limitations. One preferred example thereof is acylase "Amano" (trade name, produced by Amano Enzyme Inc.), being isolated from microorganisms (filamentous fungus belonging to aspergillus). It can be purchased easily in the commercial marketplace. As for the D-aminoacylase, one that has the ability of hydrolyzing selectively the N-acyl group in D form of the N-acyl form represented by the general formula (1) may be used without particular limitations. Specific examples thereof include D-aminoacylase described in JP-B-60-31477, in more detail, D-aminoacylase isolated from pseudomonas aminovorans NCIB 9039, pseudomonas species (P. sp.) 1158 (FERM 4632) or P. sp. 617 (FERM 4631); D-aminoacylase described in JP-B-7-83711, in more detail, D-aminoacylase isolated from alcaligenes denitrificans subspecies xylosoxidans MI-4 strain (FERM 9413) ; and the like. L- or D-aminoacylase for use in the invention may generally be utilized after purification of the enzyme, but crude enzymes, microorganism whole cells or the like may be used when they have the above mentioned selective hydrolysis ability. Further, immobilized enzymes that have been prepared by immobilizing the enzyme or microorganism whole cells to a polymer carrier or the like may also be used. The immobilized enzyme is a shaped enzyme that an enzyme, microorganism whole cells or the like is immobilized to a polymer carrier or the like in such a manner that it can be collected and recycled. It is usually manufactured by a carrier bonding method, a cross-linking polymerization method, or a gel inclusion method.

In the general formulae (1) and (2), and the formulae (C) and (D), R₁ represents an acyl group, wherein the acyl group may be any group which is a substituting carbonyl group being able to be hydrolyzed with the aforementioned L- or D-aminoacylase. Preferred examples of the acyl group include an alkanoyl group having 1-10 carbons, a benzoyl group, a halogen-substituted alkanoyl group having 1-5 carbons and a halogen-substituted benzoyl group.

Specific examples of the alkanoyl group having 1-10 carbons include a formyl group, an acetyl group, a proprionyl group, an i-butylyl group, an-pentanoyl group and an i-pentanoyl group. Among them, an alkanoyl group having 1-5 carbons is preferred and an acetyl group is particularly preferred.

Examples of the halogen atom include chlorine and bromine atoms. Specific examples of the halogen-substituted alkanoyl group having 1-5 carbons include a chloroacetyl group, a chloroproprionyl group and a chlorobutylyl group. Specific examples of the halogen-substituted benzoyl group include a chlorobenzoyl group and bromobenzoyl group.

Asymmetric hydrolysis of N-acyl-DL-erythro 3-substituted serines represented by the general formula (1) with L-aminoacylase or D-aminoacylase may be carried out, for example, as follows.

The reaction may be conducted in a solution or a suspension containing N-acyl-DL-erythro 3-substituted serines represented by the general formula (1) and L-aminoacylase or D-aminoacylase at the activating temperature of the enzyme. Any reaction solvent may be usable as long as it does not spoil the activity of the enzyme. Usually, water is preferable, which may contain a polar solvent such as alcohol in a range that does not bring about obstacles to the reaction. PH of the reaction solution varies depending on enzymes, pH being usually 4-10 and preferably around 6-10. The pH of the reaction solution is preferably adjusted in the above range with a mineral acid such as hydrochloric acid, an organic acid, an inorganic base such as alkali hydroxide, or various kinds of salts, salt of a strong acid and a weak base or salt of an inorganic strong base and a weak acid. Further, a buffer solution may be formed with various kinds of acids and bases. When the aforementioned acylase "Amano" (trade name) is used, the most preferred pH is around from 7 to 9. The reaction temperature varies depending on the activating temperature of the enzyme, the temperature being usually 0-80 °C and preferably around 20-60 °C. When the aforementioned acylase "Amano" (trade name) is used, the most preferred reaction temperature is around 30-45 °C. Since amount of an enzyme used can not be mentioned generally for depending on its activity or the like, it may be determined suitably by preliminary examinations. When a purified enzyme is used, the ratio by weight of it to N-acyl-DL-erythro 3-substituted serines is around 0.001-0.3, preferably 0.05-0.2.

Separation of the optically-active form (N-acyl-D-erythro 3-substituted serine or D-erythro 3-substituted serine) from the reaction solution may by carried out according to an ordinary method such as extraction. For example, when an unnecessary optically-active form is hydrolyzed by using L-aminoacylase, the reaction solution is made acidic with a strong acid such as hydrochloric acid, followed by extracting the targeted N-acyl-D-erythro 3-substituted serine with an organic solvent, preferably with an ester-series solvent ((C₁-C₄) alkyl ester of acetic acid such as ethyl acetate, n-propyl acetate, i-propyl acetate, butyl acetate, i-butyl acetate or t-butyl acetate) and by removing the extraction solvent from the extraction solution by a method of concentration or the like to give the targeted serine. On the other hand, when the intended optically-active form is hydrolyzed with D-aminoacylase, the targeted D-erythro 3-substituted serine may be obtained by removing the unnecessary N-acyl form through extraction with an organic solvent in the same way as described above, and then by removing the reaction solvent by a step of concentrating the reaction solution or the like.

Next, with reference to reaction schemes (1), (2), (3) and (4), synthesis steps up to N-t-butoxycarbonyl-D-erythro 3-cyclohexylserine will be explained more specifically, including a part of production steps of a raw compound. Herein, in the reaction schemes, explanation will be given by using an example in which R₁ = acetyl group. However, it may also be applicable in a similar way when R₁ is another functional group.

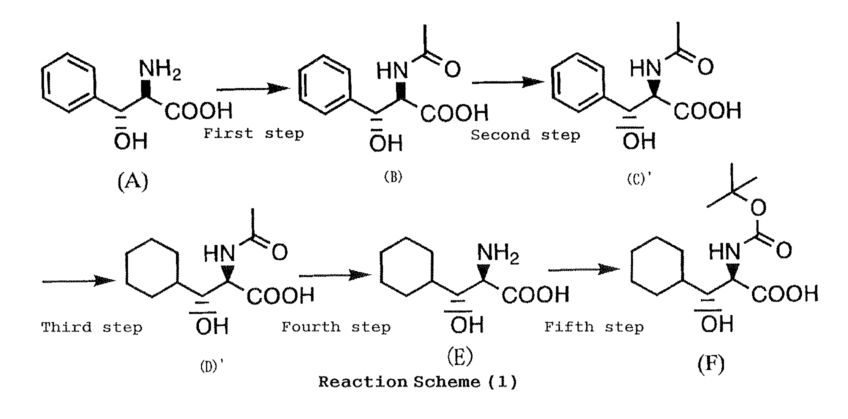

The above reaction scheme (1) illustrates the process for producing N-t-butoxycarbonyl-D-erythro 3-cyclohexylserine of formula (F), the process including a first step of acetylating DL-erythro 3-phenylserines of formula (A), a subsequent second step of hydrolyzing an unnecessary form of N-acetyl-DL-erythro 3-phenylserines represented by formula (B) with L-aminoacylase and conducting extraction with an organic solvent, or the like, to give the targeted N-acetyl-D-erythro 3-phenylserine of formula (C)', a third step in which the N-acetyl-D-erythro 3-phenylserine of formula (C)' is subjected to catalytically hydrogenating reduction with rhodium-carbon to give N-acetyl-D-erythro 3-cyclohexylserine of formula (D)', a forth step of hydrolyzing the N-acetyl-D-erythro 3-cyclohexylserine of formula (D)' with a strong acid such as hydrochloric acid to give D-erythro 3-cyclohexylserine of formula (E), and further a fifth step of converting the D-erythro 3-cyclohexylserine into the Boc form with di-t-butyldicarbonate.

In this connection, the order of the aforementioned two steps, the second step (hydrolysis and extraction) and the third step (catalytically hydrogenating reduction) may be reversed. That is, it is also possible to carry out the catalytically hydrogenating reduction first to convert a benzene ring into a cyclohexane ring followed by hydrolysis with L-aminoacylase and extraction with an organic solvent to give the targeted optically active form. However, since the rhodium-carbon for use in the catalytically hydrogenating reduction is expensive, the former order is desirable from the viewpoint of economy.

Further, in the above reaction scheme (1), it may also be possible to carry out the steps until obtaining N-acetyl-D-erythro 3-phenylserine of the formula (C)' in the second step, then to hydrolyze the obtained N-acetyl-D-erythro 3-phenylserine with a strong acid or the like to give D-erythro 3-phenylserine of formula (G) in the reaction scheme (2) below, and to employ the steps represented in the reaction scheme (2) below in the subsequent steps to produce N-t-butoxycarbonyl-D-erythro 3-cyclohexylserine of the formula (F).

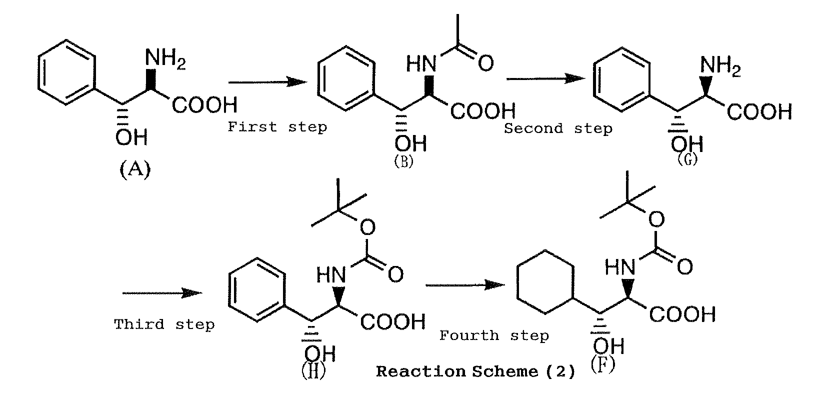

The above reaction scheme (2) illustrates the process for producing N-t-butoxycarbonyl-D-erythro 3-cyclohexylserine of the formula (F), the process including a first step of acetylating DL-erythro 3-phenylserines of the formula (A), a subsequent second step of hydrolyzing D form of N-acetyl-DL-erythro 3-phenylserines of the formula (B) with D-aminoacylase, removing the unnecessary N-acetyl form from the reaction solution by extraction with an organic solvent or the like and removing the reaction solution according to need to give D-erythro 3-phenylserine of the formula (G), a third step of converting D-erythro 3-phenylserine of the formula (G) into Boc form with di-t-butyldicarbonate to give N-t-butoxycarbonyl-D-erythro 3-phenylserine of formula (H), and a subsequent forth step of subjecting N-t-butoxycarbonyl-D-erythro 3-phenylserine of the formula (H) to catalytically hydrogenating reduction with rhodium-carbon. Also in this process, the reduction step may be conducted prior to the hydrolysis step with the enzyme.

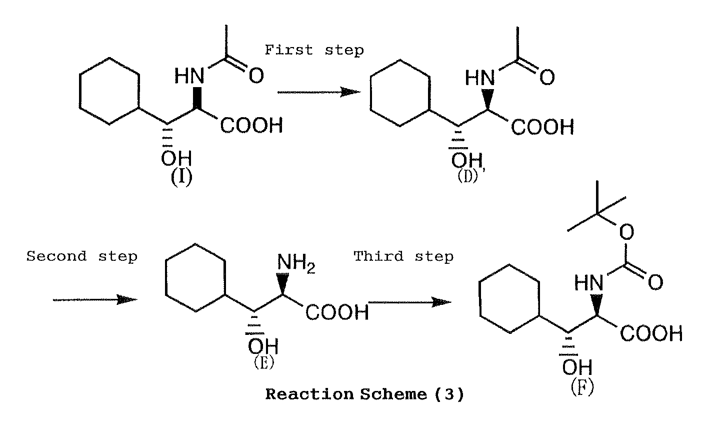

The above reaction scheme (3) illustrates the process for producing N-t-butoxycarbonyl-D-erythro 3-cyclohexylserine of the formula (F), the process including a first step of hydrolyzing the unnecessary stereo form of N-acetyl-DL-erythro 3-cyclohexylserines with L-aminoacylase and removing the same, followed by extraction ng the residue with an organic solvent, or the like, to give the targeted N-acetyl-D-erythro 3-cyclohexylserine of the formula (D)', a second step of hydrolyzing N-acetyl-D-erythro 3-cyclohexylserine of the formula (D)' to give D-erythro 3-cyclohexylserine of the formula (E), and a subsequent step 3 of converting the D-erythro 3-cyclohexylserine of the formula (E) into Boc form with di-t-butyldicarbonate.

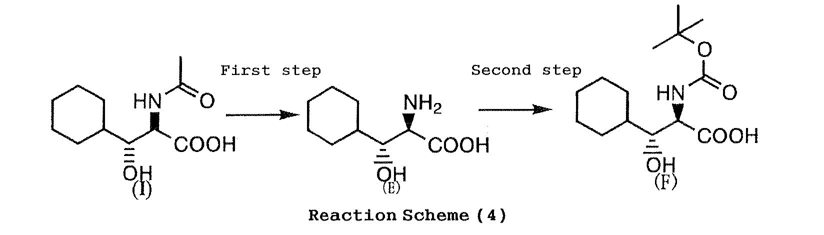

The above reaction scheme (4) illustrates the process for producing N-t-butoxycarbonyl-D-erythro 3-cyclohexylserine of the formula (F), the process including a first step of hydrolyzing D form of N-acetyl-DL-erythro 3-cyclohexylserines of the formula (I) with D-aminoacylase, removing the unnecessary N-acetyl form from the reaction solution through extraction with an organic solvent, or the like, and removing insoluble matters and the reaction solvent according to need to give D-erythro 3-cyclohexylserine of the formula (E), and a second step of converting D-erythro 3-cyclohexylserine of the formula (E) into Boc form with di-t-butyldicarbonate.

Further, each step will be explained in detail.

First, the aforementioned reaction scheme (1) is explained specifically.

DL-erythro 3-phenylserines of the formula (A) in the aforementioned reaction scheme (1) may be prepared as follows. For example, it may be obtained according to a process described in J. Am. Chem. Soc. 1953,75,238 and 1953,75,89, the process including steps of starting with ethylbenzoylacetate, going through ethylbenzoyloxyiminoacetate which is subjectred to catalytically hydrogenating reduction with palladium-carbon to give DL-erythro 3-phenylserine ethyl esters, and hydrolyzing the same. The hydrolysis step may be conducted by a general technique. For example, various techniques described in items from 550 to 563 in Theodor W Green, "Protective Group in Organic Synthesis" (John Willey & Sons, (1981)) can be mentioned.

Acetylation in the first step of the aforementioned reaction scheme (1) may be conducted according to a general technique. For example, various techniques described in items from 550 to 563 in Theodor W Green, "Protective Group in Organic Synthesis" (John Willey & Sons, (1981)) can be mentioned. That is, the targeted N-acetyl-DL-erythro 3-phenylserines represented by the formula (B) may be obtained by dissolving DL-erythro 3-phenylserines of the formula (A) in a generally used solvent such as water and dropping sodium hydroxide and acetic anhydride to the solution simultaneously.

As for a hydrolase in the second step of the aforementioned reaction scheme (1), L-aminoacylase, which hydrolyzes selectively the N-acetyl group in the L form of DL-erythro forms of the formula (B), can be mentioned. After asymmetric hydrolysis of N-acetyl-DL-erythro 3-phenylserines represented by the formula (B) using the enzyme, N-acetyl-D-erythro 3-phenylserine of the formula (C)' can be obtained.

In the case of conducting the invention by using the hydrolase, for example acylase "Amano" (trade name), N-acetyl-DL-erythro 3-phenylserines represented by the formula (B) is suspended or dissolved in an aqueous solution or a buffer solution having been adjusted to pH 4-10, preferably pH 6-10, to which the enzyme is added by a ratio by weight of preferably 0.001-0.3, preferably 0.01-0.2 to N-acetyl-DL-erythro 3-phenylserines of the formula (B), and the suspension or solution is stirred at 0-80°C, preferably 20-60°C to enable the reaction to proceed. The reaction finishes usually after from 1 hour to several days. It is preferred to keep the reaction solution pH 6-10 until the finish of the reaction.

As the aforementioned aqueous solution, an aqueous solution, in which a mineral acid such as sulfuric acid, hydrochloric acid or phosphoric acid, an organic acid such as acetic acid or citric acid, an inorganic base such as sodium hydroxide, potassium hydroxide or sodium carbonate, or a salt such as sodium acetate or sodium chloride is added, may be employed. As the buffer solution, a general buffer solution such as potassium dihydrogen phosphate-sodium hydroxide, potassium dihydrogen phosphate-sodium dihydrogen phosphate, potassium hydrogen phthalate-hydrochloric acid or glycine-sodium chloride-sodium hydroxide may be used, and no particular limitation is imposed except for one that disturbs the reaction. In addition, when the invention is conducted, an alcohol-series solvent such as methanol, ethanol, n-propanol, isopropanol, n-butanol or t-butanol, an ether-series solvent such as ether, tetrahydrofuran or dioxane, an aromatic hydrocarbon-series solvent such as benzene or toluene, a ketone-series solvent such as acetone, methyl ethyl ketone or diethyl ketone, an amine-series solvent such as triethylamine or pyridine, a polar solvent such as dimethylformamide or dimethylsulfoxide, or a surfactant such as sorbitan monopalmitate or sorbitan monolaurate may also be added according to need.

The catalytically hydrogenating reduction in the third step of the aforementioned reaction scheme (1) may be conducted by a general technique. For example, the method of catalytic hydrogen addition described in items from 333 to 448 of "Shin Jikken Kagaku Koza (New Course of Experimental Chemistry) 15 [II]" (edit. by The Chemical Society of Japan, Maruzen, 1977) may be used. That is, the targeted N-acetyl-D-erythro 3-cyclohexylserine of the formula (D)' may be obtained by dissolving N-acetyl-D-erythro 3-phenylserine of the formula (C)' in an alcohol-series solvent such as methanol, followed by conducting catalytically hydrogenating reduction using a catalyst such as, for example, rhodium-carbon as a hydrogenation catalyst. In this connection, N-acetyl-D-erythro 3-cyclohexylserine of the formula (D)' is a novel chemical substance, and is an important compound in order to obtain the targeted formula (E).

The hydrolysis reaction in the fourth step of the aforementioned reaction scheme (1) may be conducted by a general technique. For example, various techniques described in the items 550-563 of Theodor W. Green "Protective Group in Organic Synthesis" (John Willey & Sons, (1981)) can be mentioned. That is, the targeted D-erythro 3-cyclohexylserine of the formula (E) may be obtained by dissolving N-acetyl-D-erythro 3-cyclohexylserine of the formula (D)' in a generally used solvent such as water to which an acid such as hydrochloric acid is added, followed by heating and refluxing the same.

The N-t-butoxylation in the fifth step of the aforementioned reaction scheme (1) may be conducted according to a general technique. For example, various techniques described in the items 503-549 of Theodor W. Green "Protective Group in Organic Synthesis" (John Willey & Sons, (1981)) can be mentioned. That is, the targeted N-t-butoxycarbonyl-D-erythro 3-cyclohexylserine of the formula (F) may be obtained by allowing, for example, di-t-butylcarbamate to act on D-erythro 3-cyclohexylserine of the formula (E).

Next, specific explanation will be given about the aforementioned reaction scheme (2) in the production process of the invention.

The step until obtaining N-acetyl-DL-erythro 3-phenylserines of the formula (B) in the aforementioned reaction scheme (2) is the same as that described in the item of the aforementioned reaction scheme (1). Accordingly, explanation is given about steps after the first step.

As for hydrolase for use in the second step of the aforementioned reaction scheme (2), D-aminoacylase which hydrolyzes selectively the N-acetyl group of N-acetyl-D-erythro 3-phenylserine can be mentioned. D-erythro 3-phenylserine of the formula (G) may be obtained by asymmetrically hydrolyzing N-acetyl-DL-erythro 3-phenylserines represented by the formula (B) using this enzyme, followed by removing unreacted N-acetyl-L-erythro 3-phenylserine from the reaction solution through extraction with an organic solvent, or the like, and by removing the insoluble matter and reaction solvent according to need.

The asymmetric hydrolysis with the enzyme may be conducted in the same way as that in the second step of the aforementioned reaction scheme (1).

N-t-butoxylation in the third step of the aforementioned reaction scheme (2) is conducted according to the process represented in the fifth step of the aforementioned reaction scheme (1), enabling acquisition of the targeted N-t-butoxycarbonyl-D-erythro 3-phenylserine of the formula (H).

Catalytically hydrogenating reduction in the fourth step of the aforementioned reaction scheme (2) is conducted according to the process represented in the third step of the aforementioned reaction scheme (1), enabling acquisition of the targeted N-t-butoxycarbonyl-D-erythro 3-cyclohexylserine of the formula (F).

Next, specific explanation will be given about the aforementioned reaction scheme (3).

Synthesis of DL-erythro 3-cyclohexylserines of the formula (I) in the aforementioned reaction scheme (3) may be conducted as follows. According to a process, for example, described in J. Am. Chem. Soc. 1953, 75, 238 and 1953, 75, 89, ethylbenzoyloxyiminoacetate is obtained from ethylbenzoylacetate, and this is subjected to catalytically hydrogenating reduction with palladium-carbon to give DL-erythro 3-phenylserine ethyl esters, which are then subjected to hydrolysis to give DL-erythro 3-phenylserines. Further, the same are acetylated according to the same process as that in the first step of the aforementioned reaction scheme (1), which are subjected to catalytically hydrogenating reduction with rhodium-carbon in the same way as the third step of the aforementioned reaction scheme (1), enabling acquisition of the targeted DL-erythro 3-cyclohexylserines of the formula (I).

As for hydrolase in the first step of the aforementioned reaction scheme (3), L-aminoacylase which hydrolyzes selectively the acyl group of N-acetyl-L-erythro 3-cyclohexylserine can be mentioned. As the enzyme, the acylase "Amano" described above can be mentioned specifically. N-acetyl-D-erythro 3-cyclohexylserine of the formula (D)' may be obtained by subjecting N-acetyl-DL-erythro 3-cyclohexylserines represented by the formula (I) to asymmetric hydrolysis using the L-aminoacylase, extracting N-acetyl-D-erythro 3-cyclohexyl-serine with an organic solvent, and by removing the solvent according to need.

The above asymmetric hydrolysis may be conducted in the same way as that in the second step of the aforementioned reaction scheme (1).

Hydrolysis in the second step of the aforementioned reaction scheme (3) may be conducted according to the process shown in the fourth step of the aforementioned reaction scheme (1), enabling acquisition of the targeted D-erythro 3-cyclohexylserine of the formula (E).

N-t-butoxylation in the third step of the aforementioned reaction scheme (3) may be conducted according to the same process as that in the fifth step of the aforementioned reaction scheme (1), enabling acquisition of the targeted N-t-butoxycarbonyl-D-erythro 3-cyclohexylserine of the formula (F).

Next, specific explanation will be given about the aforementioned reaction scheme (4).

N-acetyl-DL-erythro 3-cyclohexylserines of the formula (I) in the aforementioned reaction scheme (4) may be obtained by the process described in the item of the aforementioned reaction scheme (3).

As for ester hydrolase in the step 1 of the aforementioned reaction scheme (4), D-aminoacylase can be mentioned. D-erythro 3-cyclohexylserine of the formula (E) may be obtained by subjecting N-acetyl-DL-erythro 3-cyclohexylserines represented by the formula (I) to asymmetric hydrolysis using this enzame, removing unnecessary N-acetyl form from the reaction solution through extraction with an organic solvent, or the like, and by removing the insoluble matter and reaction solvent according to need.

The asymmetric hydrolysis may be conducted in the same way as that in the second step of the aforementioned reaction scheme (1).

N-t-butoxylation in the second step of the aforementioned reaction scheme (4) may be conducted according to the process described in the fifth step of the aforementioned reaction scheme (1), enabling acquisition of the targeted N-t-butoxycarbonyl-D-erythro 3-cyclohexylserine of the formula (F).

In this connection, optically active erythro 3-cyclohexylserine or Boc form thereof obtained according to the process of the present application can be used to give medicines that are useful as an anti-HIV drug by employing methods described in examples 45 and 46 etc. of WO 01/40227.

### Examples

Hereinafter, the process of the invention will be explained in more detail with reference to Examples, however the invention is not limited by the Examples. In the Examples, % represents % by weight.

### Referential example 1

### Production of N-acetyl-DL-erythro 3-phenylserines (formula (B))

11.0 g of DL-erythro 3-phenylserine ethyl esters were suspended in 30 ml of water, which was heated to 40 °C. After dropping 42 ml of a 10 % aqueous solution of sodium hydroxide, the suspension was further heated to 65 °C and stirred for 2 hours. After cooling this solution to 5 °C, 4.6 g of acetic anhydride and a 1 N sodium hydroxide aqueous solution were added dropwise simultaneously while keeping the solution pH 10-13 and 10 °C or less. Then, the solution was stirred at room temperature overnight.

After adjusting the solution to pH 1 with hydrochloric acid, extraction was conducted with ethyl acetate. The obtained organic layer was washed with saturated saline, dried over magnesium sulfate, and vacuum-concentrated, to which a mixture of ethyl acetate and n-hexane (ethyl acetate:n-hexane = 19:7) was added to precipitate crystals. The obtained crystals were filtrated and then vacuum-dried to give 5.79 g of N-acetyl-DL-erythro 3-phenylserines (formula (B)).
¹H-NMR (200 MHz, CD₃OD: δ7.42-7.28 (m, 5H), δ4.97 (d, 1H) δ4.76 (d, 1H) δ1.86 (s, 3H)

### Example 1

### Production of N-acetyl-D-erythro 3-phenylserine (formula (C)')

To 2.23g of N-acetyl-DL-erythro 3-phenylserines (formula (B)) was added 100 ml of water to form a suspension, which was adjusted to pH 8 with aqueous ammonia. Inner temperature was set to 37 °C ± 5 °C by using a water bath. Thereto, 200 mg of L-aminoacylase (acylase "Amano", produced by Amano Enzyme Inc.) was added. The mixture was stirred at 37 °C ± 5 °C for 68 hours and then additional 100 mg of L-aminoacylase was added thereto, and the mixture was adjusted to pH 8 with aqueous ammonia and allowed to react for 24 hours. To the reaction solution was added acetic acid to make it pH 5, then the reaction solution was heated to 60 °C and stirred for 1 hour. Subsequently, insoluble matter was filtrated and removed. After vacuum-concentrating the filtrate, 1 N aqueous hydrochloric acid was used to make it pH 3. To the solution was added sodium chloride and the solution was extracted with ethyl acetate. The obtained organic layer was washed with saturated saline, dried over magnesium sulfate, and vacuum-concentrated to give 0.9 g of N-acetyl-D-erythro 3-phenylserine (formula (C)').
¹H-NMR (200 MHz, CD₃OD: δ4.68 (d, 1H:), δ3.49 (dd, 1H), δ2.03 (s, 3H), δ1.99-0.94 (m, 11H)

### Example 2

### (1) Production of N-acetyl-D-erythro 3-cyclohexylserine (formula (D)')

0.9 g of N-acetyl-D-erythro 3-phenylserine (formula (C)') obtained in Example 1 was dissolved in 20 ml of methanol. 5% rhodium-carbon was added thereto and the obtained mixture was stirred, under 0.9 Mpa hydrogen pressure, at 50 °C for 4 hours. The insoluble matter such as the catalyst was removed from the obtained reaction solution through filtration, and the filtrate was vacuum-concentrated to give 1.0 g of N-acetyl-D-erythro- cyclohexylserine (formula (D)').

### (2) Production of D-erythro 3-cyclohexylserine (formula (E))

1.0 g of N-acetyl-D-erythro 3-cyclohexylserine (formula (D)') was suspended in a mixture of 10 ml of water and 3 ml of hydrochloric acid. The obtained suspension was heated and refluxed for 3 hours, thereafter, vacuum-concentrated, and to the obtained residue was added 40 ml of water, and the obtained solution was made to pH 7 using 28 % aqueous ammonia to precipitate crystals. The obtained crystals were filtrated to give 0.46 g of D-erythro 3-cyclohexylserine (formula (E)).

### (3) Production of N-t-butoxycarbonyl-D-3-erythro-cyclohexylserine (formula (F))

0.46 g of D-erythro 3-cyclohexylserine (formula (E)) was suspended in 10 ml of water. To the suspension was added 0.11 g of sodium hydroxide and the suspension was cooled to 10 °C or less. Then, to the suspension was added 10 ml of acetone and added dropwise a solution prepared by dissolving 0.54 g of di-t-butyldicarbonate in 10 ml of acetone, and the obtained solution was stirred at room temperature overnight. The reaction solution was made acidic with hydrochloric acid, which was extracted with ethyl acetate. The obtained organic layer was washed with saturated saline, dried over magnesium sulfate, and vacuum-concentrated. To the residue was added n-heptane to precipitate crystals, and then filtrated to give 0.65 g of N-butoxycarbonyl-D-erythro 3-cyclohexylserine (formula (F)).
¹H-NMR (200 MHz, CD₃OD: δ4.33 (d, 1H), δ3.72 (dd, 1H), δ1.05 (s, 9H), δ2.07-0.94 (m, 11H)

### Industrial Applicability

The process of the invention for producing N-acyl optically active erythro 3-substituted serines represented by the above general formula (2) that utilizes asymmetric hydrolysis with L-aminoacylase or D-aminoacylase is, compared with a chemical synthesis process or a process using an optical resolution agent, inexpensive, simple and of high-efficiency and, further, capable of reduce the amount of an organic solvent used to result in low environmental load. Thus, it is an industrially useful production process.

## Claims

1. A process for producing N-acyl-D-erythro 3-substituted serine represented by the general formula (2): 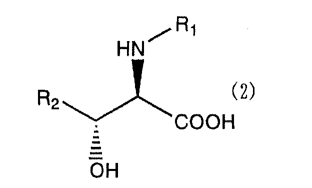 (in the formula (2), R₁ represents an acyl group and R₂ means a phenyl group or a cyclohexyl group), **characterized by** allowing L-aminoacylase having the ability of hydrolyzing selectively N-acyl-L-erythro 3-substituted serines to act on N-acyl-DL-erythro 3-substituted serines represented by the general formula (1) to conduct asymmetrical hydrolysis: 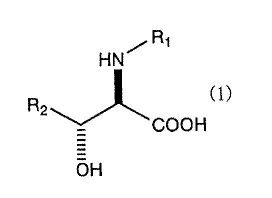 (in the formula (1), R₁ and R₂ represent the same meanings as those in the formula (2)).

2. The process according to claim 1, wherein the acyl group is an alkanoyl group having 1-10 carbons, a benzoyl group, a halogen-substituted alkanoyl group having 1-5 carbons or a halogen-substituted benzoyl group.

3. The process according to claim 1, wherein R₁ is an alkanoyl group having 1-5 carbons.

4. The process according to claim 3, wherein R₁ is an acetyl group.

5. A process of producing D-erythro 3-substituted serine represented by the general formula (3): 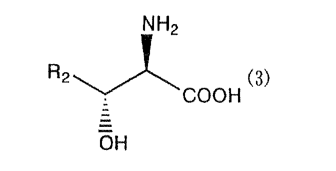 (in the formula (3), R₂ means a phenyl group or a cyclohexyl group), **characterized by** allowing D-aminoacylase having the ability of hydrolyzing selectively N-acyl-D-erythro 3-substituted serine to act on N-acyl-DL-erythro 3-substituted serines represented by the general formula (1) to conduct asymmetrical hydrolysis: 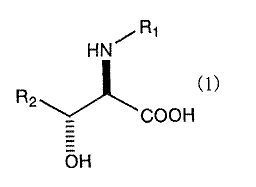 (in the formula (1), R₁ represents an acyl group, and R₂ means a phenyl group or a cyclohexyl group).

6. The process according to claim 5, wherein the acyl group is an alkanoyl group having 1-10 carbons, a benzoyl group, a halogen-substituted alkanoyl group having 1-5 carbons or a halogen-substituted benzoyl group.

7. The process according to claim 5, wherein R₁ is an alkanoyl group having 1-5 carbons.

8. The process according to claim 7, wherein R₁ is an acetyl group.

9. A process for producing D-erythro 3-cyclohexylserine represented by the following formula (E): 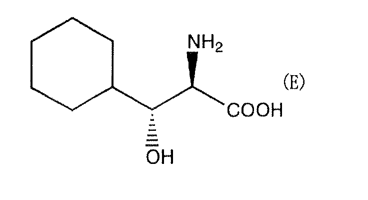 **characterized by** comprising the steps of:
subjecting N-acyl-D-erythro 3-phenylserine represented by the following formula (C) obtained by the production process according to in any one of claims 1-4: 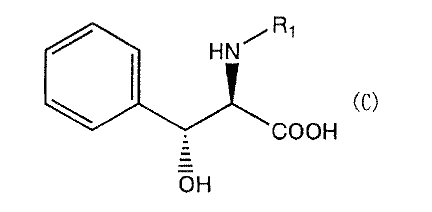 (in the formula (C), R₁ represents an acyl group) to catalytically hydrogenating reduction with rhodium-carbon to give N-acyl-D-erythro 3-cyclohexylserine represented by the following formula (D): 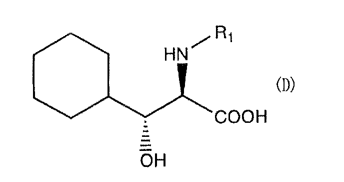 (in the formula (D), R₁ represents the same meaning as that in the formula (C)); and
hydrolyzing a compound of the formula (D).

10. A process for producing N-t-butoxycarbonyl-D-erythro 3-cyclohexylserine represented by the following formula (F): 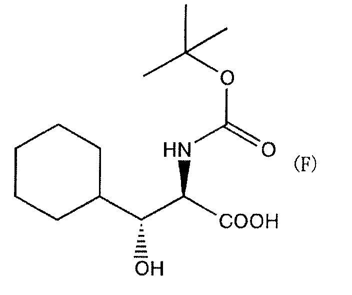 comprising the steps of:
converting D-erythro 3-phenylserine represented by the following formula (G) obtained by the process according to any one of claims 5 to 8: 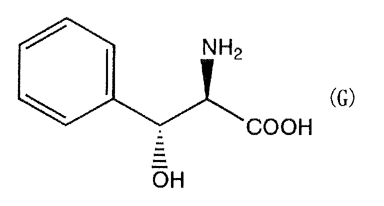 into Boc form with di-t-butyldicarbonate to give N-t-butoxycarbonyl-D-erythro 3-phenylserine represented by the following formula (H): 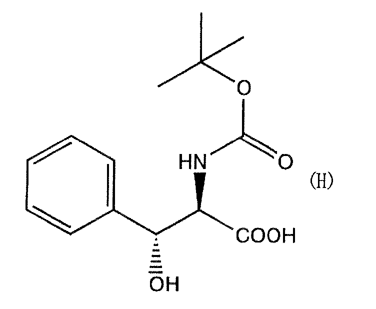 and
subjecting a compound of the formula (H) to catalytically hydrogenating reduction with rhodium-carbon.

11. N-acetyl-D-erythro 3-cyclohexylserine represented by the following formula (D)':
